(19) Europäisches Patentamt
European Patent Office
Office européen des brevets

(11) Numéro de publication: **0 351 255 B1**

(12) **FASCICULE DE BREVET EUROPEEN**

(45) Date de publication de fascicule du brevet: **13.10.93**

(51) Int. Cl.[5]: **C07D 401/06**, A61K 31/47

(21) Numéro de dépôt: **89400306.0**

(22) Date de dépôt: **03.02.89**

(54) **Dérivés de [(pipéridinyl-4)méthyl]-2 tétrahydro-1,2,3,4 isoquinoléine, leur préparation et leur application en thérapeutique.**

(30) Priorité: **12.07.88 FR 8809447**

(43) Date de publication de la demande:
**17.01.90 Bulletin 90/03**

(45) Mention de la délivrance du brevet:
**13.10.93 Bulletin 93/41**

(84) Etats contractants désignés:
**AT BE CH DE ES FR GB GR IT LI LU NL SE**

(56) Documents cités:
**FR-A- 1 580 180**
**FR-A- 2 035 063**
**FR-M- 7 607**

(73) Titulaire: **SYNTHELABO**
**22, Avenue Galilée**
**F-92350 Le Plessis Robinson(FR)**

(72) Inventeur: **George, Pascal**
**19, rue des Ouatre Vents**
**F-78730 St Arnoult en Yvelines(FR)**
Inventeur: **Sevrin, Mireille**
**73, rue Raymond Losserand**
**F-75014 Paris(FR)**
Inventeur: **Maloizel, Christian**
**21, rue du Plateau**
**F-92190 Meudon(FR)**
Inventeur: **Tixidre, Arlette**
**Bt 1 - Résidence les Fonds Fanettes**
**F-91190 Gif/Yvette(FR)**
Inventeur: **Froissant, Jacques**
**Cidex 981 Brevainville**
**F-41160 Moree(FR)**

(74) Mandataire: **Ludwig, Jacques et al**
**SYNTHELABO**
**Service Brevets**
**22, avenue Galilée**
**F-92352 LE PLESSIS ROBINSON CEDEX (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

La présente demande de brevet a pour objet des dérivés de [(pipéridinyl-4)méthyl]-2 tétrahydro-1,2,3,4 isoquinoléine, leur préparation et leur application en thérapeutique.

Elle s'apparente à la demande de brevet français N° 8711288 du 7 Août 1987. Cette dernière a pour objet des composés répondant à la formule générale suivante

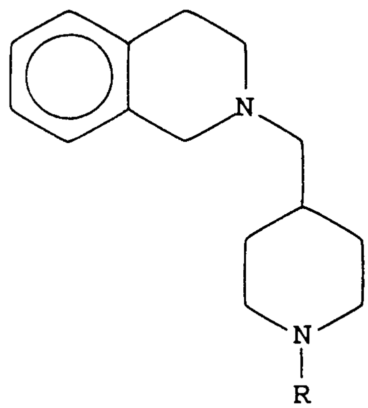

dans laquelle R représente soit un atome d'hydrogène, soit un groupe méthyle, soit un groupe benzyle éventuellement substitué par un atome de chlore ou un groupe méthyle ou méthoxy, soit encore un groupe phénéthyle.

Les composés de la présente demande répondent à la formule générale (I)

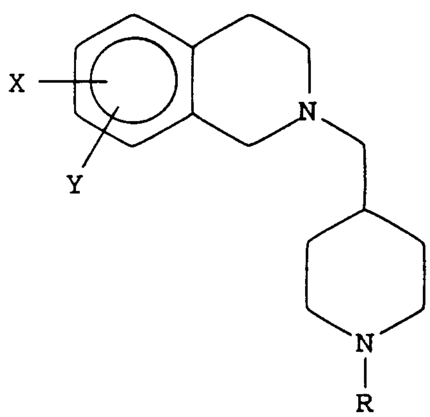

(I)

dans laquelle

X représente un atome d'hydrogène ou un groupe méthyle ou méthoxy,

Y représente un atome d'halogène ou un groupe méthyle ou méthoxy, et

R représente un groupe de formule générale -Z-R′ dans laquelle Z représente un groupe $-CH_2-$ ou un groupe -CO- et R′ représente un groupe phényle portant éventuellement de un à trois substituants choisis parmi les atomes d'halogènes et les groupes trifluorométhyle, $(C_1-C_3)$alkyle linéaires ou ramifiés et $(C_1-C_3)$-alcoxy linéaires ou ramifiés.

Ils peuvent se présenter à l'état de bases libres ou de sels d'addition à des acides.

Conformément à l'invention on peut préparer les composés de formule générale (I) par un procédé illustré par le schéma 1 donné ci-après.

On prépare d'abord un composé de formule générale (I) où Z représente un groupe -CO- en faisant réagir une tétrahydro-1,2,3,4 isoquinoléine de formule générale (II) (dans laquelle X et Y sont tels que définis ci-dessus), avec un tosylate de formule générale (III) (dans laquelle Tos représente un groupe tosyle et R′ est tel que défini ci-dessus), soit en l'absence, soit en présence d'un solvant inerte tel que le diméthylformamide, le toluène ou le xylène, à une température de 20 à 150 °C, et éventuellement en présence d'une base organique, telle qu'une amine tertiaire, ou minérale, telle qu'un carbonate ou hydrogénocarbonate alcalin.

On obtient ainsi un composé de formule générale (Ia) qui correspond à la formule générale (I) lorsque Z représente un groupe -CO-.

Si l'on désire préparer un composé de formule générale (I) où Z représente un groupe $-CH_2-$, on réduit ensuite le composé de formule générale (Ia) au moyen d'un hydrure simple ou complexe de bore ou d'aluminium, par exemple l'hydrure de lithium et d'aluminium, l'hydrure d'aluminium, le complexe diborane/tétrahydrofuranne ou le complexe diborane/sulfure de méthyle, ou tout moyen équivalent, dans un solvant éthéré tel que l'éther diéthylique, le tétrahydrofuranne ou le dioxanne, à une température de 20 à 100 °C.

On obtient ainsi un composé de formule générale (Ib) qui correspond à la formule générale (I) lorsque Z représente un groupe $-CH_2-$.

Schéma 1

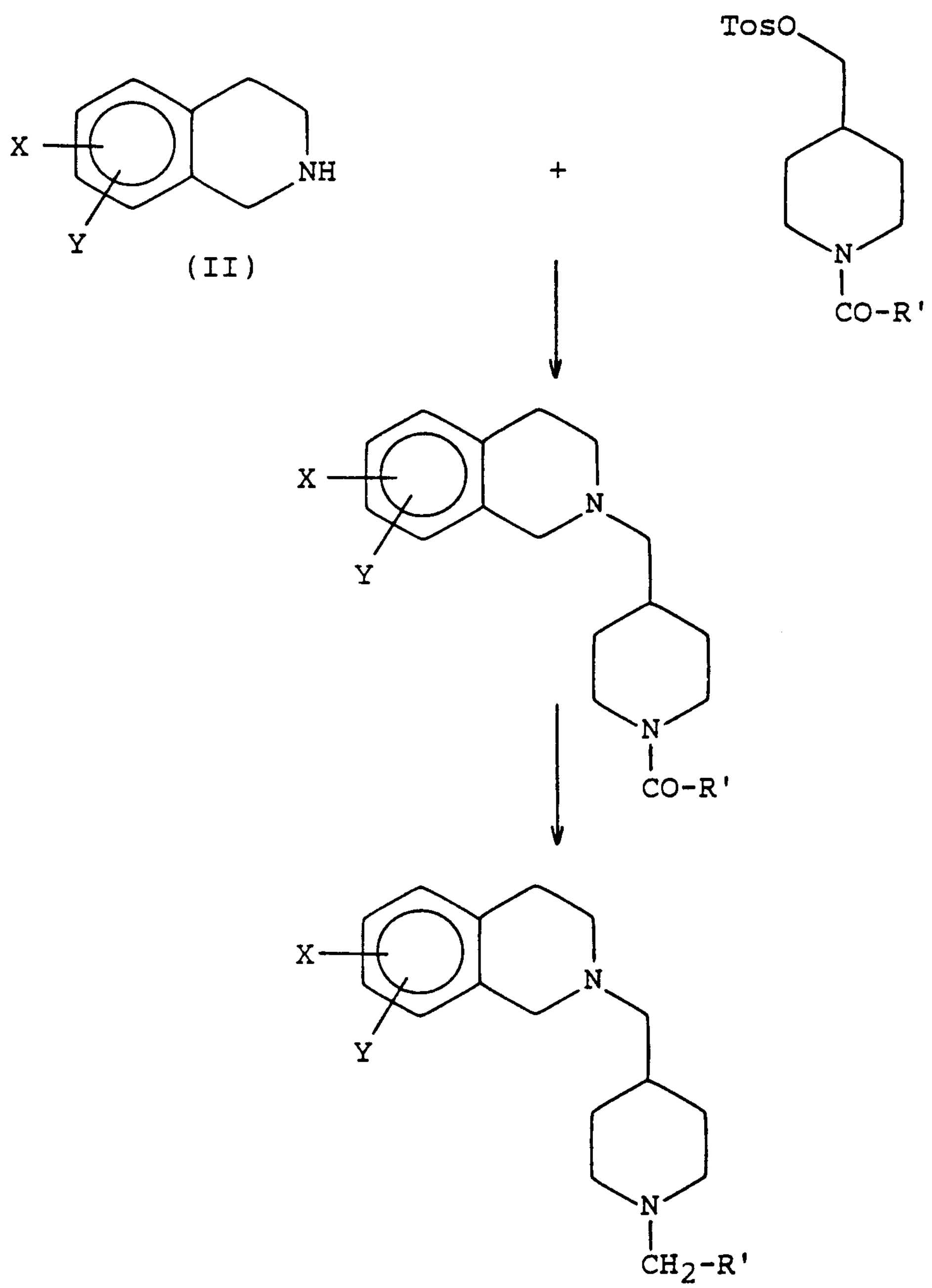

(II)

(III)

(Ia)

(Ib)

Schéma 2

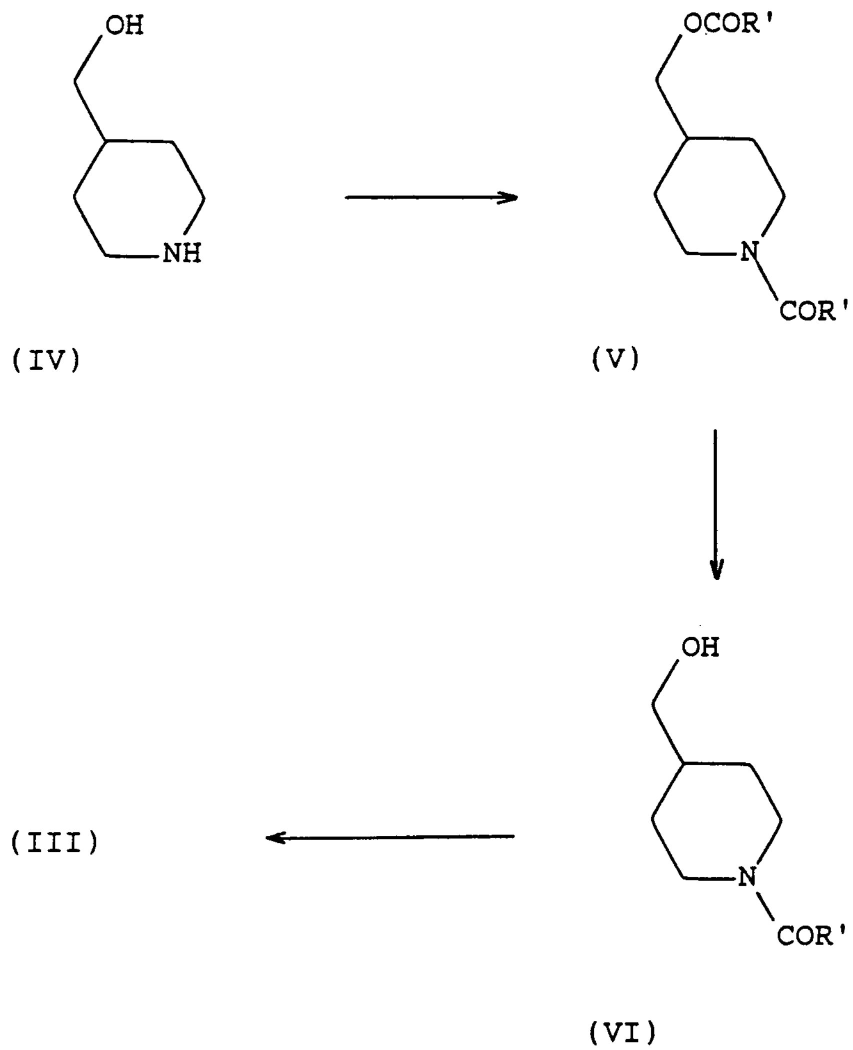

(IV) (V)

(III)

(VI)

Les tétrahydro-1,2,3,4 isoquinoléines de formule générale (II) peuvent être obtenues par un procédé tel que celui décrit par D. J. Sall et G. L. Grunewald dans J. Med. Chem., 1987, 30, 2208-2216, ou par toute méthode analogue.

Les tosylates de formule générale (III) peuvent être préparés selon une méthode illustrée par le schéma 2 qui précède. On fait réagir le pipéridine-4-méthanol de formule (IV) avec un chlorure d'acide de formule générale R'COCl (dans laquelle R' est tel que défini ci-dessus), dans un solvant inerte tel qu'un solvant chloré, à une température de 20 à 80 °C. On obtient ainsi un ester-amide de formule générale (V), qu'on saponifie , par exemple au moyen d'hydroxyde de sodium ou de potassium, dans un solvant alcoolique aliphatique inférieur, de préférence l'éthanol, pour obtenir l'alcool de formule générale (VI), dont on prépare finalement le tosylate au moyen de chlorure de tosyle, dans un milieu basique tel que la pyridine.

Le pipéridine-4-méthanol de formule (IV) peut être obtenu par exemple par réduction de pipéridine-4-carboxylate d'éthyle au moyen d'hydrure de lithium et aluminium, ou encore par une telle réduction de benzyl-1 pipéridine-4-carboxylate d'éthyle suivie d'une hydrogénolyse catalytique sous pression.

Par ailleurs, la demande de brevet principale et les demandes de premier et deuxième certificats d'addition N° 8718342 et 8805129 décrivent divers procédés de préparation de composés de formule générale (I) où X et Y représenteraient chacun un atome d'hydrogène ; il va de soi que ces procédés, dans leurs principes, sont également applicables pour la préparation des composés substitués de la présente invention.

Les exemples suivants illustrent en détail la préparation de quelques composés selon l'invention. Les microanalyses élémentaires et les spectres IR et RMN confirment les structures des produits obtenus.

Les numéros indiqués entre parenthèses dans les titres des exemples correspondent à ceux du tableau donné plus loin.

Exemple 1 (Composé N° 16)

Diméthoxy-5,8 [[[(méthyl-3 phényl)carbonyl]-1 pipéridinyl-4]méthyl]-2 tétrahydro-1,2,3,4 isoquinoléine, fumarate.

1.1. Pipéridine-4-méthanol.

Dans un ballon tricol de 4 l muni d'un système d'agitation mécanique et d'un réfrigérant on introduit 28,5 g (0,75 mole) d'hydrure de lithium et aluminium et 1,2 l de tétrahydrofuranne. On ajoute à la suspension obtenue 117,9 g (0,75 mole) de pipéridine-4-carboxylate d'éthyle en solution dans 1,2 l de tétrahydrofuranne, et on agite le mélange pendant 6h à 20° C. On le refroidit à 0° C, puis on l'hydrolyse en ajoutant successivement 22 ml d'eau, 22 ml d'hydroxyde de sodium 1N et 46 ml d'eau. On agite le mélange 30mn à 20° C, on le filtre, on lave le précipité au tétrahydrofuranne puis à l'éther. On évapore les solvants sous pression réduite, et on obtient 84,4 g d'une huile qu'on utilise telle quelle dans l'étape suivante.

1.2. Méthyl-3 benzoate de [(méthyl-3 benzoyl)-1 pipéridinyl-4]méthyle.

Dans un ballon tricol de 3 l on introduit, sous atmosphère d'argon, 42,25 g (0,367 mole) de pipéridine-4-méthanol, 430 ml de dichloro-1,2 éthane, et on ajoute 82 g (0,81 mole) de triéthylamine puis 125,2 g (0,81 mole) de chlorure de méthyl-3 benzoyle. On chauffe le mélange au reflux pendant 4h30, on ajoute encore 8,2 g (0,08 mole) de triéthylamine et 12,5 g (0,08 mole) de chlorure de méthyl-3 benzoyle, et on chauffe le mélange pendant encore 3h.
On le filtre, on lave les sels au dichloro-1,2 éthane, on évapore le filtrat sous pression réduite, on dissout le résidu dans de l'acétate d'éthyle, on lave la solution avec une solution aqueuse saturée de chlorure de sodium, on évapore le solvant sous pression réduite, et on recristallise le résidu dans un mélange 1/1 d'alcool isopropylique/acétate d'éthyle. On obtient 80 g d'un solide blanc. Point de fusion : 80-83° C.

1.3. (Méthyl-3 benzoyl)-1 pipéridine-4-méthanol.

A une solution de 80 g (0,23 mole) de méthyl-3 benzoate de [(méthyl-3 benzoyl)-1 pipéridinyl-4]méthyle dans 400 ml d'éthanol, on ajoute une solution de 12,76 g (0,23 mole) d'hydroxyde de potassium dans 75 ml d'éthanol et 75 ml d'eau. On agite le mélange à 20° C pendant 3h, on évapore le solvant sous pression réduite et on extrait la phase aqueuse à l'acétate d'éthyle. On lave la phase organique avec de l'eau puis avec une solution aqueuse saturée de chlorure de sodium, et on la séche sur sulfate de magnésium. On évapore le solvant sous pression réduite et on obtient 53 g d'alcool qu'on utilise tel quel dans l'étape suivante.

1.4. Méthyl-4 benzènesulfonate de [(méthyl-3 benzoyl)-1 pipéridinyl-4]méthyle.

A une solution de 52 g (0,22 mole) de (méthyl-3 benzoyl)-1 pipéridine-4-méthanol dans 100 ml de pyridine on ajoute 53,3 g (0,28 mole) de chlorure de méthyl-4 benzènesulfonyle dans 60 ml de pyridine. On agite le mélange à 20° C pendant 4h, puis on le verse dans de la glace. On extrait la phase au dichlorométhane, on lave la phase organique avec une solution aqueuse 10N d'acide chlorhydrique et on la séche sur sulfate de magnésium. On évapore les solvants sous pression réduite et on obtient 70 g de solide blanc. Point de fusion : 68-70° C.

1.5. Diméthoxy-5,8 [[[(méthyl-3 phényl)carbonyl]-1 pipéridinyl-4]méthyl]-2 tétrahydro-1,2,3,4 isoquinoléine, fumarate.

Sous atmosphère d'argon on chauffe pendant 20h un mélange de 3,86 g (0,02 mole) de diméthoxy-5,8 tétrahydro-1,2,3,4 isoquinoléine et 8,5 g de méthyl-4 benzènesulfonate de [(méthyl-3 benzoyl)-1 pipéridinyl-4]méthyle. On ajoute au mélange du dichlorométhane puis de l'ammoniaque concentrée, on sépare la phase organique, on la lave deux fois à l'eau, on la sèche sur sulfate de magnésium, on la filtre et on évapore le solvant sous pression réduite. On obtient un résidu huileux qu'on reprend avec de l'éther, on

sépare une fraction insoluble par filtration, on évapore l'éther et on purifie le résidu par chromatographie sur colonne de silice en éluant avec un mélange 98/2 de dichlorométhane/méthanol. On isole ainsi 3,3 g de base pure.
On en dissout 1,6 g dans le minimum d'éthanol, on ajoute 0,45 g d'acide fumarique dissous dans 60 ml d'éthanol, on évapore l'éthanol et on recristallise le résidu dans l'alcool isopropylique. On isole finalement 1,3 g de fumarate. Point de fusion : 156-158 ° C.

Exemple 2 (Composé N ° 17)

Diméthoxy-5,8 [[[(méthyl-3 phényl)méthyl]-1 pipéridinyl-4]méthyl]-2 tétrahydro-1,2,3,4 isoquinoléine, difumarate.

A une solution de 1,6 g (0,0038 mole) de diméthoxy-5,8 [[[(méthyl-3 phényl)carbonyl]-1 pipéridinyl-4]-méthyl]-2 tétrahydro-1,2,3,4 isoquinoléine dans 30 ml d'éther on ajoute 0,29 g (0,0078 mole) d'hydrure de lithium et aluminium, on agite le mélange pendant 3h à température ambiante, on la refroidit avec un bain de glace et on l'hydrolyse avec 2,4 ml de soude 1N. On filtre le mélange et on évapore le filtrat, ce qui fournit 1,4 g de base. On dissout cette dernière dans le minimum d'éthanol, on ajoute 0,82 g d'acide fumarique en solution dans 100 ml d'éthanol, on évapore l'éthanol et on recristallise le résidu dans l'alcool isopropylique. On isole finalement 1,5 g de difumarate. Point de fusion : 198-201 ° C.

Le tableau ci-aprés illustre les structures chimiques et les propriétés physiques de quelques composés selon l'invention.
Dans la dernière colonne "(d)" indique la décomposition du composé au point de fusion.

Tableau

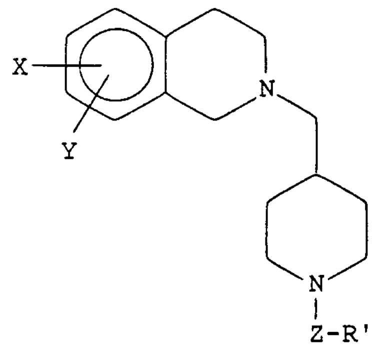

| N° | X | Y | Z | R' | Sel(*) | F(°C) |
|---|---|---|---|---|---|---|
| 1 | H | $CH_3$-5 | CO | $C_6H_5$- | HCl | 211-212 |
| 2 | H | $CH_3$-5 | $CH_2$ | $C_6H_5$- | difum. | 194-196 |
| 3 | H | $CH_3$-5 | CO | Cl-3-$C_6H_4$- | HCl | 214-216 |
| 4 | H | $CH_3$-5 | $CH_2$ | Cl-3-$C_6H_4$- | difum. | 206-208 |
| 5 | H | $CH_3$-5 | CO | $C_2H_5O$-3-$C_6H_4$- | HCl | 179-182 |
| 6 | H | $CH_3O$-5 | CO | $C_6H_5$- | HCl | 230(d) |
| 7 | H | $CH_3O$-5 | $CH_2$ | $C_6H_5$- | difum. | 188-190 |
| 8 | H | $CH_3O$-5 | CO | Cl-3-$C_6H_4$- | fum. | 168-170 |
| 9 | H | $CH_3O$-6 | CO | $C_6H_5$- | HCl | 183-185 |
| 10 | H | $CH_3O$-8 | CO | $C_6H_5$- | HCl | 205-215(d) |
| 11 | H | $CH_3O$-8 | $CH_2$ | $C_6H_5$- | difum. | 198-201 |
| 12 | $CH_3O$-5 | $CH_3O$-8 | CO | $C_6H_5$- | HCl | 240-246(d) |
| 13 | $CH_3O$-5 | $CH_3O$-8 | $CH_2$ | $C_6H_5$- | difum. | 193-197 |
| 14 | $CH_3O$-5 | $CH_3O$-8 | CO | Cl-3-$C_6H_4$- | fum. | 151-152 |
| 15 | $CH_3O$-5 | $CH_3O$-8 | $CH_2$ | Cl-3-$C_6H_4$- | difum. | 200-202 |
| 16 | $CH_3O$-5 | $CH_3O$-8 | CO | $CH_3$-3-$C_6H_4$- | fum. | 156-158 |
| 17 | $CH_3O$-5 | $CH_3O$-8 | $CH_2$ | $CH_3$-3-$C_6H_4$- | difum. | 198-201 |
| 18 | $CH_3O$-5 | $CH_3O$-8 | CO | $C_2H_5O$-3-$C_6H_4$- | ½fum. | 134-135 |
| 19 | $CH_3O$-5 | $CH_3O$-8 | $CH_2$ | $C_2H_5O$-3-$C_6H_4$- | difum. | 178-180 |
| 20 | $CH_3O$-5 | $CH_3O$-8 | $CH_2$ | $CF_3$-3-$C_6H_4$- | difum. | 193-203(d) |
| 21 | $CH_3O$-6 | $CH_3O$-7 | CO | $C_6H_5$- | fum. | 138-139 |
| 22 | H | Cl-6 | CO | $C_6H_5$- | HCl | 222-225 |
| 23 | H | Cl-6 | $CH_2$ | $C_6H_5$- | difum. | 136-139 |
| 24 | H | Cl-8 | CO | $C_6H_5$- | HCl | 198-200 |

(*) HCl, fum., difum. et ½fum. désignent respectivement un chlorhydrate, un fumarate, un difumarate et un hémifumarate.

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type $5\text{-}HT_{1A}$.Les composés déplacent dans l'hippocampe du rat un ligand spécifique marqué, la $[^3H]$-hydroxy-8 dipropylamino-2 tétraline (désignée ci-après par "$[^3H]$-8-OH-DPAT") décrite par Gozlan et coll., Nature,

(1983), 305, 140-142.

Les animaux utilisés sont des rats mâles Sprague-Dawley de 160 à 200 g. Aprés décapitation on en préléve le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4 °C, en les centrifugeant à chaque fois à 48000xg et en remettant le culot en suspension pendant 10 mn dans du tampon frais refroidi. Finalement on met le dernier culot en suspension dans le tampon pour arriver à une concentration de 100 mg de tissu de départ par ml de tampon à 50 mM.

On laisse ensuite incuber à 37 °C pendant 10 mn.

La liaison avec la [$^3$H]-8-OH-DPAT est déterminée par incubation de 10 $\mu$l de suspension de membranes dans un volume final de 1 ml de tampon contenant 10 $\mu$M de pargylline.

Aprés l'incubation on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 $\mu$M. A une concentration de 1 nM de [$^3$H]-8-OH-DPAT la liaison spécifique représente de 70 à 80% de la radioactivité totale recupérée sur le filtre.

Pour chaque concentration de composés étudié on détermine le pourcentage d'inhibition de la liaison avec la [$^3$H]-8-OH-DPAT, puis la concentration $CI_{50}$, concentration qui inhibe 50% de la liaison.

Pour les composés de l'invention les $CI_{50}$ se situent entre 0,001 et 0,3 $\mu$M.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,003 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes.

Pour les composés de l'invention les $DE_{50}$ se situent entre 0,003 et 3 mg/kg par la voie intraveineuse.

Les résultats des essais montrent que les composés de formule générale (I) possédent, in vitro, une grande affinité et une sélectivité pour les récepteurs sérotoninergiques de type 5-$HT_{1A}$. In vivo ils montrent une activité agoniste, agoniste partielle, ou antagoniste au niveau de ces récepteurs.

Les composés de l'invention peuvent donc être utilisés pour le traitement des maladies et affections impliquant les récepteurs sérotoninergiques de type 5-$HT_{1A}$ de façon directe ou indirecte, notamment pour le traitement des états dépressifs, des états d'anxiété, des troubles du sommeil, pour la régulation de la prise de nourriture, et pour le traitement vasculaires, cardiovasculaires ou cérébrovasculaires tels que l'hypertension ou la migraine.

A cet effet ils peuvent être présentés sous toutes formes appropriées à leur administration par voie orale ou parentérale, associés à tous excipients convenables, et dosés pour permettre une posologie journalière de 1 à 1000 mg.

**Revendications**

**Revendications pour les Etats contractants suivants : AT, BE, CH, DE, FR, GB, IT, LI, LU, NL, SE**

**1.** Composés de formule générale (I)

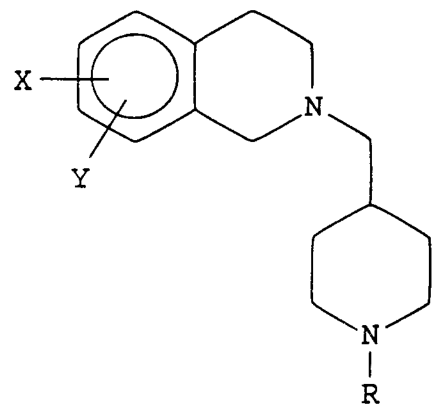

(I)

dans laquelle
X représente un atome d'hydrogène ou un groupe méthyle ou méthoxy,
Y représente un atome d'halogène ou un groupe méthyle ou méthoxy, et
R représente un groupe de formule générale -Z-R' dans laquelle Z représente un groupe $-CH_2-$ ou un groupe -CO- et R' représente un groupe phényle portant éventuellement de un à trois substituants choisis parmi les atomes d'halogènes et les groupes trifluorométhyle, $(C_1-C_3)$alkyle linéaires ou ramifiés et $(C_1-C_3)$alcoxy linéaires ou ramifiés,
ainsi que leurs sels d'addition à des acides.

**2.** Procédé de préparation de composés selon la revendication 1, caractérisé en ce qu'on fait réagir une tétrahydro-1,2,3,4 isoquinoléine de formule générale (II)

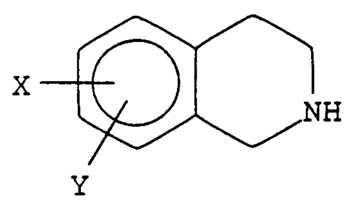

(II)

(dans laquelle X et Y sont tels que définis dans la revendication 1), avec un tosylate de formule générale (III)

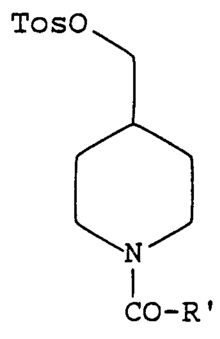

(III)

(dans laquelle Tos représente un groupe tosyle et R′ est tel que défini dans la revendication 1), soit en l'absence, soit en présence d'un solvant inerte tel que le diméthylformamide, le toluène ou le xylène, à une température de 20 à 150 °C, et éventuellement en présence d'une base organique, telle qu'une

amine tertiaire, ou minérale, telle qu'un carbonate ou hydrogénocarbonate alcalin, obtenant ainsi un composé de formule générale (Ia)

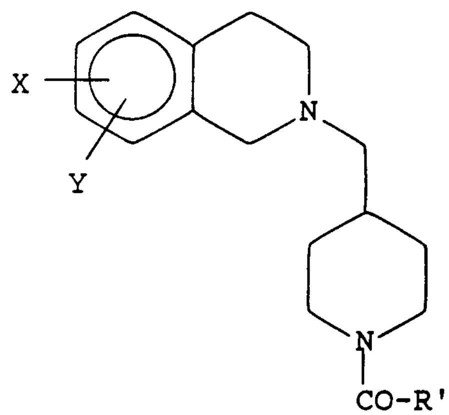

(Ia)

puis, si l'on désire préparer un composé de formule générale (I) où Z représente un groupe $-CH_2-$, on réduit ensuite le composé de formule générale (Ia) au moyen d'un hydrure simple ou complexe de bore ou d'aluminium, tel que l'hydrure de lithium et d'aluminium, l'hydrure d'aluminium, le complexe diborane/tétrahydrofuranne ou le complexe diborane/sulfure de méthyle, dans un solvant éthéré tel que l'éther diéthylique, le tétrahydrofuranne ou le dioxanne, à une température de 20 à 100 °C.

**3.** Composition pharmaceutique caractérisée en ce qu'elle contient un composé selon la revendication 1 associé à un excipient pharmaceutique.

**Revendication pour les Etats contractants suivants: ES, GR**

**1.** Procédé de préparation de composés de formule générale (I)

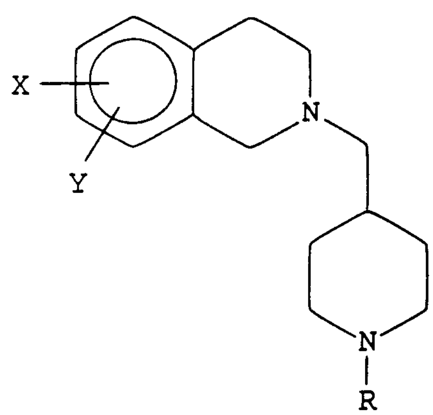

(I)

dans laquelle
X représente un atome d'hydrogène ou un groupe méthyle ou méthoxy,
Y représente un atome d'halogène ou un groupe méthyle ou méthoxy, et
R représente un groupe de formule générale -Z-R′ dans laquelle Z représente un groupe $-CH_2-$ ou un groupe -CO- et R′ représente un groupe phényle portant éventuellement de un à trois substituants choisis parmi les atomes d'halogènes et les groupes trifluorométhyle, $(C_1-C_3)$alkyle linéaires ou ramifiés et $(C_1-C_3)$alcoxy linéaires ou ramifiés,
procédé caractérisé en ce qu'on fait réagir une tétrahydro-1,2,3,4 isoquinoléine de formule générale (II)

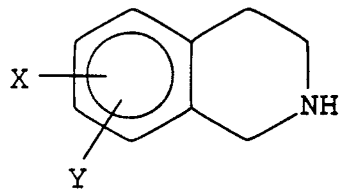

(II)

(dans laquelle X et Y sont tels que définis ci-dessus), avec un tosylate de formule générale (III)

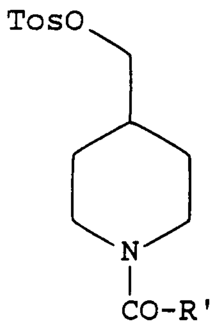

(III)

(dans laquelle Tos représente un groupe tosyle et R′ est tel que défini ci-dessus), soit en l'absence, soit en présence d'un solvant inerte tel que le diméthylformamide, le toluène ou le xylène, à une température de 20 à 150 ° C, et éventuellement en présence d'une base organique, telle qu'une amine tertiaire, ou minérale, telle qu'un carbonate ou hydrogénocarbonate alcalin,
obtenant ainsi un composé de formule générale (Ia)

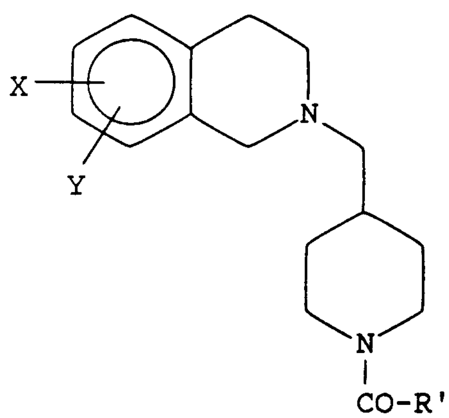

(Ia)

puis, si l'on désire préparer un composé de formule générale (I) où Z représente un groupe $-CH_2-$, on réduit ensuite le composé de formule générale (Ia) au moyen d'un hydrure simple ou complexe de bore ou d'aluminium, tel que l'hydrure de lithium et d'aluminium, l'hydrure d'aluminium, le complexe diborane/tétrahydrofuranne ou le complexe diborane/sulfure de méthyle, dans un solvant éthéré tel que l'éther diéthylique, le tétrahydrofuranne ou le dioxanne, à une température de 20 à 100 ° C.

## Claims

**Claims for the following Contracting States: AT, BE, CH, DE, FR, GB, IT, LI, LU, NL, SE**

**1.** Compounds of general formula (I)

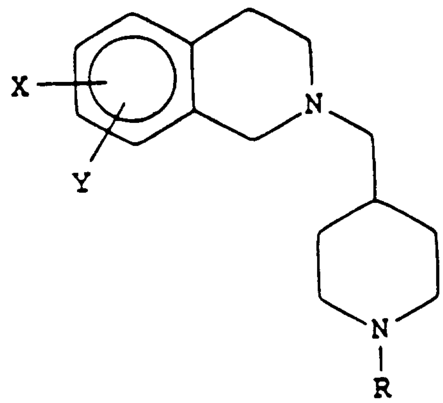

(I)

in which
X represents a hydrogen atom or a methyl or methoxy group,
Y represents a halogen atom or a methyl or methoxy group, and
R represents a group of general formula -Z-R' in which Z represents a $-CH_2-$ group or a -CO- group and R' represents a phenyl group optionally carrying one to three substituents selected from among halogen atoms and trifluoromethyl, linear or branched $(C_1-C_3)$ alkyl, and linear or branched $(C_1-C_3)$ alkoxy groups,
and their acid addition salts.

**2.** Process for the preparation of compounds according to Claim 1, characterised in that a 1,2,3,4-tetrahydroisoquinoline of general formula (II)

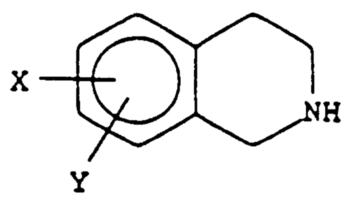

(II)

(in which X and Y are as defined in Claim 1) is reacted with a tosylate of general formula (III)

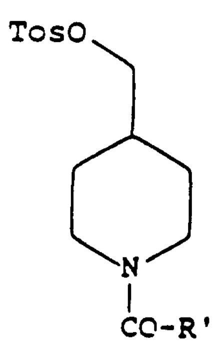

(III)

(in which Tos represents a tosyl group and R' is as defined in Claim 1), either in the absence or in the presence of an inert solvent such as dimethylformamide, toluene or xylene, at a temperature of 20 to 150 °C, and optionally in the presence of an organic base, such as a tertiary amine, or an inorganic base, such as an alkali metal carbonate or bicarbonate, thus obtaining a compound of general formula

(Ia)

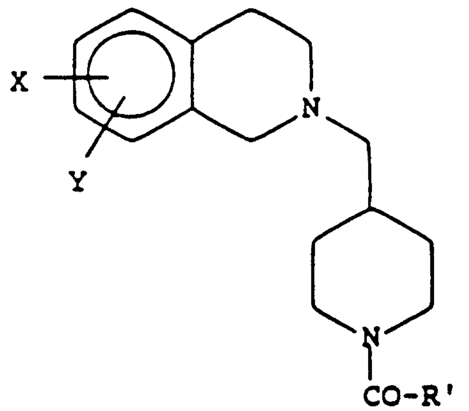

(Ia)

then, if it is required to prepare a compound of general formula (I) where Z represents a $-CH_2-$ group, the compound of general formula (Ia) is subsequently reduced with a simple or complex boron or aluminium hydride, such as lithium aluminium hydride, aluminium hydride, the diborane/tetrahydrofuran complex or the diborane/methyl sulphide complex, in an ethereal solvent such as diethyl ether, tetrahydrofuran or dioxane, at a temperature of 20 to 100 °C.

**3.** Pharmaceutical composition characterised in that it contains a compound according to Claim 1 made up with a pharmaceutical excipient.

**Claim for the following Contracting States : ES, GR**

**1.** Process for the preparation of compounds of general formula (I)

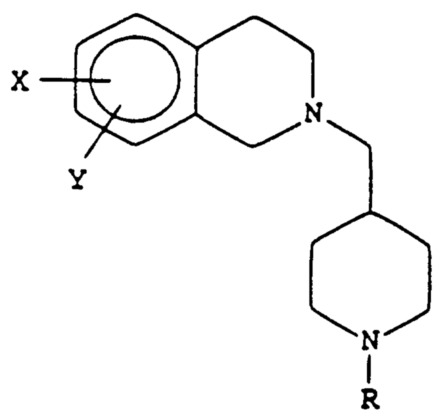

(I)

in which
X represents a hydrogen atom or a methyl or methoxy group,
Y represents a halogen atom or a methyl or methoxy group, and
R represents a group of general formula -Z-R' in which Z represents a $-CH_2-$ group or a -CO- group and R' represents a phenyl group optionally carrying one to three substituents selected from among halogen atoms and trifluoromethyl, linear or branched ($C_1$-$C_3$) alkyl, and linear or branched ($C_1$-$C_3$) alkoxy groups,
process characterised in that a 1,2,3,4-tetrahydroisoquinoline of general formula (II)

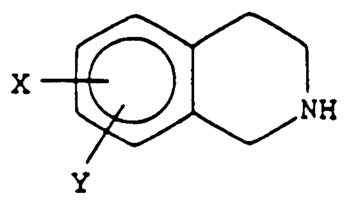

(II)

(in which X and Y are as defined above) is reacted with a tosylate of general formula (III)

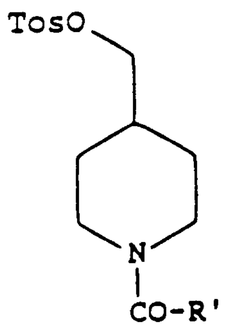

(III)

(in which Tos represents a tosyl group and R' is as defined above), either in the absence or in the presence of an inert solvent such as dimethylformamide, toluene or xylene, at a temperature of 20 to 150 °C, and optionally in the presence of an organic base, such as a tertiary amine, or an inorganic base, such as an alkali metal carbonate or bicarbonate,
thus obtaining a compound of general formula (Ia)

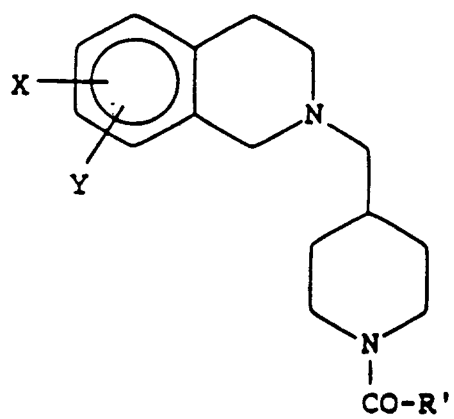

(Ia)

then, if it is required to prepare a compound of general formula (I) where Z represents a $-CH_2-$ group, the compound of general formula (Ia) is subsequently reduced with a simple or complex boron or aluminium hydride, such as lithium aluminium hydride, aluminium hydride, the diborane/tetrahydrofuran complex or the diborane/methyl sulphide complex, in an ethereal solvent such as diethyl ether, tetrahydrofuran or dioxane, at a temperature of 20 to 100 °C.

**Patentansprüche**
**Patentansprüche für folgende Vertragsstaaten : AT, BE, CH, DE, FR, GB, IT, LI, LU, NL, SE**

1. Verbindungen der allgemeinen Formel (I)

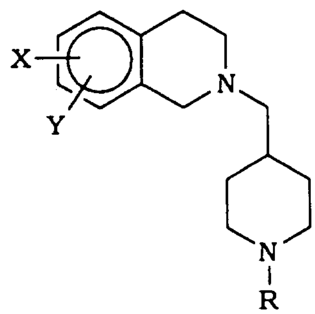

(I)

in der X ein Wasserstoffatom oder eine Methyl- oder Methoxygruppe.
Y ein Halogenatom oder eine Methyl- oder Methoxygruppe und
R eine Gruppe der allgemeinen Formel -Z-R', worin Z eine Gruppe $-CH_2-$ oder eine Gruppe -CO- und R' eine Phenylgruppe, die gegebenenfalls ein bis drei Substituenten ausgewählt aus Halogenatomen und Trifluormethylgruppen, geradkettigen oder verzweigten ($C_1$-$C_3$)-Alkylgruppen und geradkettigen oder verzweigten ($C_1$-$C_3$)-Alkoxygruppen substituiert ist, darstellen, bedeuten, sowie deren Additionssalze mit Säuren.

2. Verfahren zur Herstellung der Verbindungen nach Anspruch 1, **dadurch gekennzeichnet**, daß man ein 1,2,3,4-Tetrahydro-isochinolin der allgemeinen Formel (II)

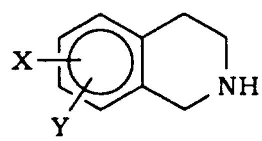

(II)

(in der X und Y die in Anspruch 1 angegebenen Bedeutungen besitzen), mit einem Tosylat der allgemeinen Formel (III)

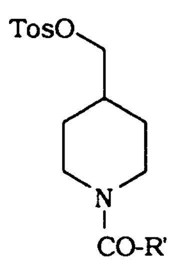

(III)

(worin Tos eine Tosylgruppe darstellt und R' die in Anspruch 1 angegebenen Bedeutungen besitzt) entweder in Abwesenheit oder in Gegenwart eines inerten Lösungsmittels, wie Dimethylformamid, Toluol oder Xylol, bei einer Temperatur von 20 bis 150 °C und gegebenenfalls in Gegenwart einer organischen Base, wie eines tertiären Amins, oder einer anorganischen Base, wie eines Alkalimetallcarbonats oder -hydrogencarbonats umsetzt,
so daß man eine Verbindung der allgemeinen Formel (Ia)

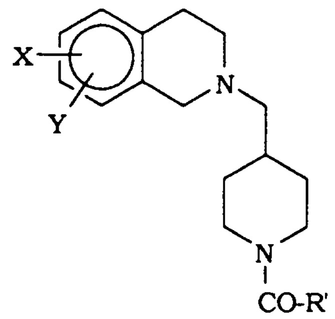

(Ia)

erhält, wonach man, wenn man eine Verbindung der allgemeinen Formel (I) herstellen will, worin Z eine Gruppe $-CH_2-$ darstellt, anschließend die Verbindung der allgemeinen Formel (Ia) mit Hilfe eines einfachen Hydrids oder eines komplexen Hydrids von Bor oder Aluminium, wie Lithiumaluminiumhydrid, Aluminiumhydrid, den Diboran/Tetrahydrofuran-Komplex oder den Diboran/Methylsulfid-Komplex, in einem Etherlösungsmittel, wie Diethylether, Tetrahydrofuran oder Dioxan, bei einer Temperatur von 20 bis 100 °C reduziert.

**3.** Pharmazeutische Zubereitung, **dadurch gekennzeichnet**, daß sie eine Verbindung nach Anspruch 1 in Kombination mit einem pharmazeutischen Trägermaterial enthält.

**Patentanspruch für folgende Vertragsstaaten : ES, GR**

**1.** Verfahren zur Herstellung von Verbindungen der allgemeinen Formel (I)

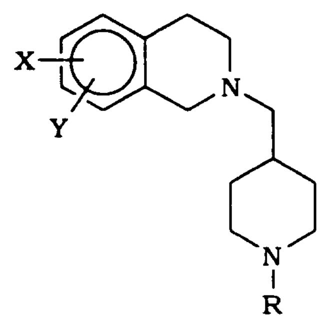

(I)

in der X ein Wasserstoffatom oder eine Methyl- oder Methoxygruppe,
Y ein Halogenatom oder eine Methyl- oder Methoxygruppe und
R eine Gruppe der allgemeinen Formel -Z-R', worin Z eine Gruppe $-CH_2-$ oder eine Gruppe -CO- und R' eine Phenylgruppe, die gegebenenfalls ein bis drei Substituenten ausgewählt aus Halogenatomen und Trifluormethylgruppen, geradkettigen oder verzweigten $(C_1-C_3)$-Alkylgruppen und geradkettigen oder verzweigten $(C_1-C_3)$-Alkoxygruppen substituiert ist, darstellen, bedeuten, **dadurch gekennzeichnet**, daß man ein 1,2,3,4-Tetrahydro-isochinolin der allgemeinen Formel (II)

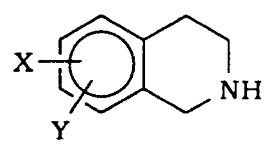

(II)

(in der X und Y die in Anspruch 1 angegebenen Bedeutungen besitzen), mit einem Tosylat der allgemeinen Formel (III)

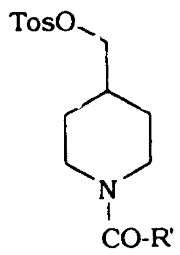

(III)

(worin Tos eine Tosylgruppe darstellt und R' die in Anspruch 1 angegebenen Bedeutungen besitzt) entweder in Abwesenheit oder in Gegenwart eines inerten Lösungsmittels, wie Dimethylformamid, Toluol oder Xylol, bei einer Temperatur von 20 bis 150 °C und gegebenenfalls in Gegenwart einer organischen Base, wie eines tertiären Amins, oder einer anorganischen Base, wie eines Alkalimetallcarbonats oder -hydrogencarbonats umsetzt,
so daß man eine Verbindung der allgemeinen Formel (Ia)

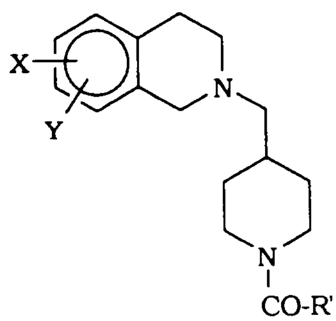

(Ia)

erhält, wonach man, wenn man eine Verbindung der allgemeinen Formel (I) herstellen will, worin Z eine Gruppe $-CH_2-$ darstellt, anschließend die Verbindung der allgemeinen Formel (Ia) mit Hilfe eines einfachen Hydrids oder eines komplexen Hydrids von Bor oder Aluminium, wie Lithiumaluminiumhydrid, Aluminiumhydrid, den Diboran/Tetrahydrofuran-Komplex oder den Diboran/ Methylsulfid-Komplex, in einem Etherlösungsmittel, wie Diethylether, Tetrahydrofuran oder Dioxan, bei einer Temperatur von 20 bis 100 °C reduziert.